# EUROPEAN PATENT SPECIFICATION

(11) **EP 1 916 975 B1**
(45) Date of publication and mention of the grant of the patent: **16.11.2011**
(21) Application number: 06779073.3
(22) Date of filing: 03.08.2006
(51) Int. Cl.: A61F 9/00

(54) **APPARATUS FOR APPLYING MEDICATION TO THE EYE**
VORRICHTUNG ZUM VERABREICHEN EINES MEDIKAMENTS INS AUGE
APPAREIL POUR LA DISTRIBUTION D'UN MEDICAMENT SUR L'OEIL

(30) Priority: 05.08.2005 GB 0516106; 17.02.2006 GB 0603205
(43) Date of publication of application: 07.05.2008
(73) Proprietor: Ian Harrison Associates, Saundersfoot Dyfed SA69 9AU (GB)
(72) Inventor: HARRISON, Ian, Dyfed SA69 9AU (GB)
(74) Representative: Davies, Gregory Mark
(86) International application number: PCT/GB2006/002895
(87) International publication number: WO 2007/017636

(56) References cited:
- DE-A1- 2 362 147
- DE-A1- 10 250 473
- US-A- 3 016 898
- US-A- 3 446 209
- US-A- 4 183 355

## Description

The present invention relates generally to medication applied to the eye, and more specifically to apparatus for applying medication to the eye.

Medication in the form of eye drops is commonly used to treat many ocular ailments, wherein the patient is supplied with eye drops to be administered directly into the eye at regular intervals.

Providing the patient is of a reasonable age and the treatment only requires a quantity of the medication to be administered to the eye in general, this task is not so much of a problem. However, if the patient is infirm for example, and/or if it is required to administer a precise quantity of the medication to a specific area of the eye, this task can become significantly more difficult and demanding. In addition, contact between the dispenser and the eyeball should be avoided at all costs to avoid the possibility of infection or injury.

It would therefore be of great benefit if there were a way of making such ocular self-treatment easier and safer, and it is an object of the present invention to provide an applicator for enabling liquid medication to be safely and conveniently applied to the correct part of the eye without the possibility of contact between the eye and the dispenser from which said liquid medication is dispensed.

D1 (US4183355) discloses a frame for therapeutic self administration of medication to the eyes and comprises a transparent right angularly related slide with normal slots therethrough for reception of guide adjustment lock means. These means are for the selective positioning of the slides, and for locking the slides in position and for receiving the medication dropper in alignment with a target area of the eye. The present invention provides an improved arrangement.

In accordance with the present invention, there is an applicator as defined in claim 1.

Thus, the above-mentioned object is achieved by providing an applicator, that can be held over a user's eye, thereby shielding the eye, and whereby the eye drops can be administered very precisely via the support and opening in the shield, without the possibility of contact between the eye and the medication dispenser.

In a preferred embodiment, the support is adjustably mounted in the opening such that it's location relative to the user's eye can be selectively adjusted. In one exemplary embodiment, the opening may comprise one or more elongate channels in which the support can be slidably mounted. Means may be provided for immobilising the support at a desired position within said opening. Such means may comprise means for generating mutual friction between said support and a contact edge of said opening.

In one exemplary embodiment, the opening may be provided in a plate portion, rotatably mounted in or on said shield portion, such that said opening can be rotated with respect to a generally central, substantially vertical axis, allowing location of the support relative to the users eye at any point within a circle swept by the length of the opening to be achieved.

Beneficially, at least one retaining strap is provided for retaining said shield portion over said user's eye, said retaining strap beneficially being connected to the applicator at substantially diametrically opposing edge parts thereof. The strap may comprise an elasticated retaining strap, fashioned as a single piece connected at each side of the shield portion or in two pieces with a means for releasably joining the two.

Preferably, said shield portion is comprised of a rigid or semi-rigid transparent material.

Beneficially, a rim is provided on said shield portion, configured to facilitate a comfortable interface between said applicator and the socket around said user's eye.

Preferably, said opening comprises two elongate channels in communication with each other; the channels beneficially intersect, possibly at respective mid-points thereof. The channels may be substantially orthogonal relative to each other.

Beneficially, said support has a neck portion that is suitably received in said opening. The neck portion is beneficially provided with an outer circumferential groove with which the contact edges of the opening engage.

At least one of the channels preferably further comprises an enlarged opening therein. The enlarged opening of the channel is preferably located at an end of a channel, and is shaped and configured to enable the outer circumferential groove of said support to pass therethrough. This enables cleaning of the support, and provides a further advantage that the applicator may be worn at night for example. The shape of the circumferential groove preferably matches that of the enlarged opening such that the groove must be at the same configuration as the opening to release therefrom.

Preferably, an opening in said support contains means is provided, such as helical wall parts, to regulate the flow of medication passing from said support to said user's eye.

Beneficially, said support is generally frusto-conical in shape, being beneficially tapered inwardly from an upper opening to a lower neck portion.

These and other aspects of the present invention will be apparent from, and elucidated with reference to, the embodiments described herein.

Embodiments of the present will now be described, by way of example only, and with reference to the accompanying drawings, in which:
Figure 1 is a plan view of an applicator according to a first exemplary embodiment of the present invention;
Figure 2 is a side view of the applicator of Figure 1;
Figure 3 is a plan view of an applicator according to a second exemplary embodiment of the present invention; and
Figure 4 is a side view of the applicator of Figure 3.

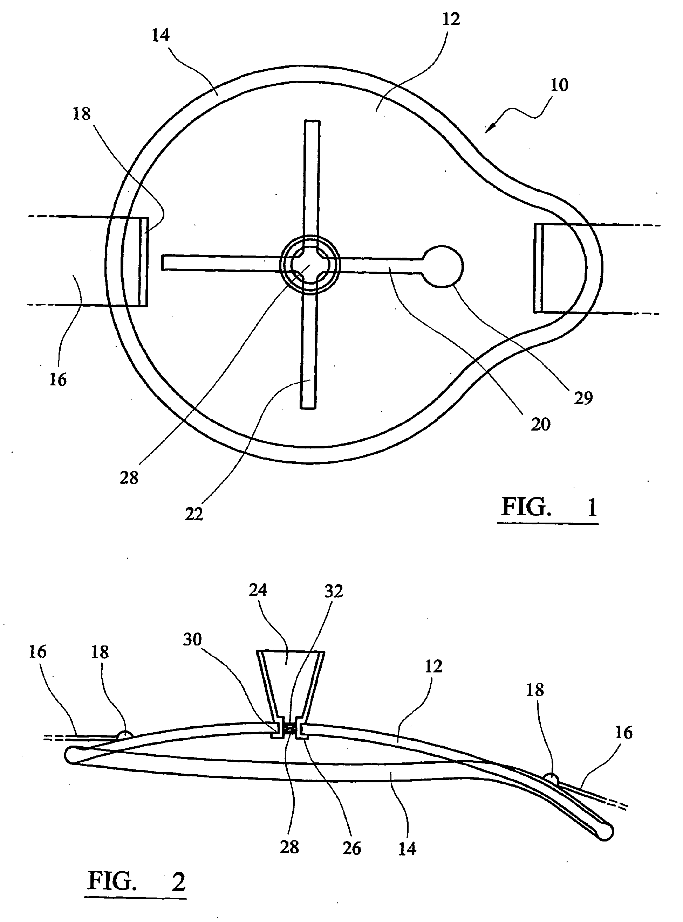

Referring to Figures 1 and 2 of the accompanying drawings, a first exemplary embodiment of an applicator 10 according to the invention comprises a generally convex shield portion 12 shaped and configured in accordance with the contours of a human eye socket such that, when the applicator 10 is positioned for use over an eye, the rim 14 sits comfortably within the recess defined by the eye socket. The rim 14 may be integrally moulded with the shield portion 12, or it may comprise a separate piece of the same or a different material, that is adhered to the perimeter of the shield portion 12. The shield portion 12 is preferably made of a rigid or semi-rigid, preferably transparent material such as acrylic, such that a user can see through it, at least to a certain extent, when the applicator 10 is positioned for use over the eye.

An elasticated retaining strap 16 is connected to the applicator 10, at diametrically opposing edge parts thereof, by respective fastening sections 18, that are anchored to the shield portion 12. This retaining strap 16 allows a user to secure the applicator 10 over the eye by engaging the strap 16, possibly around the back of the head, leaving both the users hands free.

The convex shield portion 12 defines two elongate, intersecting, orthogonal openings 20, 22 through its surface at an essentially central position, defining a substantially cross-shaped channel. A generally circular aperture 28 is defined at the point of intersection of the openings 20,22.

A support 24 is retained in, and extends upwardly from, the openings 20, 22, such that it is capable of being slidably moved to any point along the channel 20, 22. The support 24 is generally conical, inwardly tapered from the upper opening to a lower neck portion. The neck portion defines an outer circumferential groove within which the edges of the channel 20, 22 are retained, the groove being defined between the lower and of the conical portion of the support 24 and a lower lip 26, which retains the support in place in the channel 20, 22 but does not restrict its lateral movement along it.

The outer circumferential groove provided at the neck portion 30 of the support 24 may be provided with a rubber or similar outer covering, such that relative movement between the neck portion 30 and the contact edges of the channel 20, 22, will require a reasonable amount of force to effect and when the support 24 is positioned in a desirable position within the channel 20, 22 it will be retained there by mutual friction unless it is manually moved with sufficient force to overcome the friction.

The function of the support 24 is to receive the dispensing nozzle of an eye-drop container and guide the medication dispensed therefrom through, via an aperture 28, onto the users eyes. As such, the position of the support 24 within the channel defined by the intersecting, elongate openings 20, 22 dictates the location on an eye that a drop of medication will fall, whilst ensuring that the eye-drop container can never contact the eyeball itself.

The aperture 28 that allows the drop to pass defines a passageway that regulates the flow of liquid passing through it and is shaped and configured to reduce 'splash back'. A suitable structure 13 helical wall parts 32 enabling liquid to pass therethrough but preventing accidental damage to the edge by the dispensing nozzle of the eye drop container or the liquid contacting the eyeball at excess velocity.

The channels 20, 22 further define a widened portion 29 at an end of one of the openings, and as an exemplary embodiment having sufficient diameter to enable the lower lip 26 to pass therethrough. Although in use this is not desirable, in certain circumstances this may be extremely beneficial. For example, when the support 24 needs to be cleaned, it may be washed out without soaking the entire applicator 10. Additionally, if different eye medications are required, then there is a reduced chance of contamination due to the ease of cleaning. A second advantage of the inclusion of this feature is that the applicator 10 may also be used as an eye guard for a user to sleep in for example, without fear that the support 24 may become tangled in bed.

In a preferred embodiment, the widened portion is shaped to correspond to the shape of the lower lip 26 such that the support 24 must be rotated to the correct rotation that enables the support to release from the widened portion 29, which prevents accidental release.

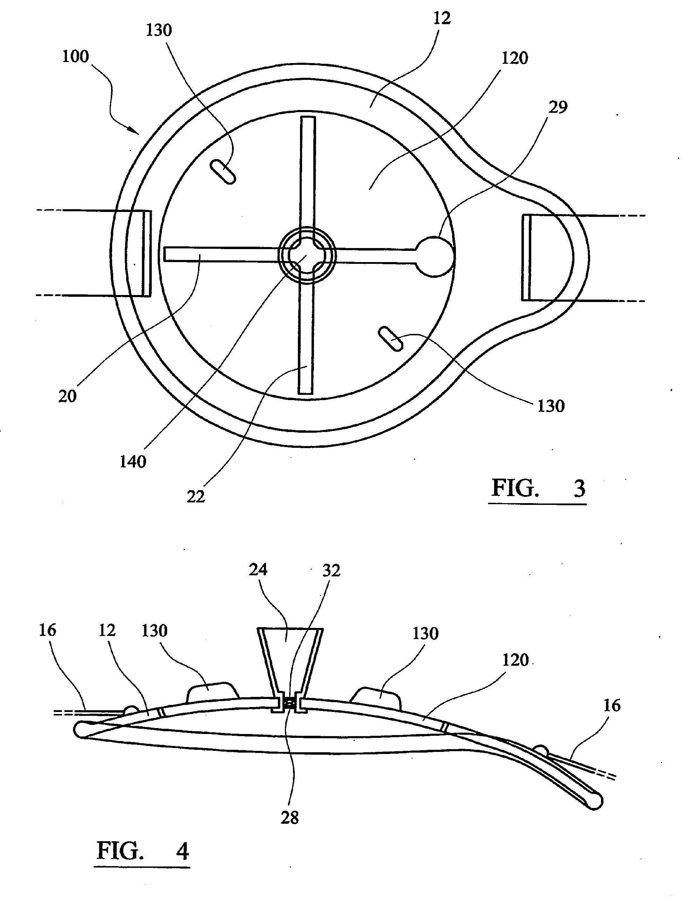

Referring now to Figures 3 and 4, an applicator 10b according to a second exemplary embodiment of the present invention is similar in many respects to that of the first exemplary embodiment, as defined above, but with means for rotating the channels 20, 22 about their central axis (being the centre-point of intersection), 140. The rotating means comprises a generally circular section 120 of the shield portion 12 being rotatably mounted on the main body of the shield portion 12, such that it can be manually rotated. A number of small raised portions 130 may be provided on the circular section 120 to facilitate convenient manual rotation thereof. These raised portions 130 may be integrally moulded or fixed to the rotatably mounted circular section 120.

The substantially cross shaped channel 20, 22 is formed in the rotatably mounted circular section 120 contains both channels 20, 22 and as such, any applicable point on the users eyeball, within the swept circumference defined by the end points of the channels 20, 22 (neglecting the radius of the section of the support 24 that passes through the channel 20, 22) can be attained by moving the support 24 along the channel 20, 22, then rotating the rotatably mounted circular section 120. It will be appreciated that it is not essential to have such a configuration of channels 20, 22 as an equally satisfactory result could be attained by, for example, a single channel defining a radius of the rotatably mounted circular section 120, or any other suitable configuration.

It should be noted that the above-mentioned embodiment illustrates rather than limits the invention, and that those skilled in the art will be capable of designing many alternative embodiments without departing from the scope of the invention as defined by the appended claims. In the claims, any reference signs placed in parentheses shall not be construed as limiting the claims. The word "comprising" and "comprises", and the like, does not exclude the presence of elements or steps other than those listed in any claim or the specification as a whole. The singular reference of an element does not exclude the plural reference of such elements and vice-versa. The invention may be implemented by means of hardware comprising several distinct elements. In a device claim enumerating several means, several of these means may be embodied by one and the same item of hardware. The mere fact that certain measures are recited in mutually different dependent claims does not indicate that a combination of these measures cannot be used to advantage.

## Claims

1. An applicator (10) for applying liquid medication to a user's eye, the applicator (10) comprising a support and a shield portion (12) for application over said user's eye, wherein said shield portion (12) comprises an opening (20, 22) for receiving the support (24) the support being arranged to receive the dispensing nozzle of an eye-drop container, **characterized in that** said medication can be dispensed into the support and wherein said opening (20, 22) is configured to guide and release said medication from said support (24) into said user's eye, and further **characterised in that** an opening in said support contains means to regulate the flow of medication passing from said support to user's eye.

2. An applicator (10) according to claim 1, wherein said support (24) is adjustably mounted in said opening such that its location relative to the user's eye can be selectively adjusted.

3. An applicator (10) according to claim 1 or 2, wherein said opening (20, 22)may comprise one or more elongate channels (20, 22) in which the support can be slidably mounted.

4. An applicator (10) according to any of claims 1 to 3, wherein means may be provided for immobilising the support at a desired position within said opening (20, 22) and optionally wherein said means may comprise means for generating mutual friction between said support and a contact edge of said opening.

5. An applicator (10) according to any preceding claim, wherein said opening (20, 22) may be provided in a plate portion, rotatably mounted in or said shield portion, such that said opening (20, 22) can be rotated with respect to a generally central, substantially vertical axis, allowing location of the support (24) relative to the user's eye at any point within a circle swept by the length of the opening to be achieved, and/or wherein at least one retaining strap (6) is provided for retaining said shield portion (12 over said user's eye, said retaining strap (16) being connected to the applicator (10) at substantially diametrically opposing edge parts thereof.

6. An applicator (10) according to claim 5, wherein said strap (16) compries an elasticated retaining strap, fashioned as a single piece connected at each side of the shield portion or in two pieces with a means for releasably joining the two.

7. An applicator according to any of claims 1 to 6, wherein said shield portion (12) is comprised of a rigid or semi-rigid transparent material.

8. An applicator (10) according to any of claims 1 to 7, wherein a rim (14) is provided on said shield portion (12), configured to facilitate a comfortable interface between said applicator (10) and the socket around said user's eye.

9. An applicator (10) according to any of claims 1 to 8, wherein said opening (20, 22) comprises two elongate channels (20, 22) in communication with each other, and optionally wherein said channels intersect at respective mid-points thereof; and optionally wherein said channels are substantially orthogonal relative to each other.

10. An applicator according to any of claims 1 to 9, wherein said support (24) has a neck portion (30) that is suitably received in said opening.

11. An applicator (10) according to claim 10, wherein said neck portion (30) is provided with an outer circumferential groove with which the contact edges of the opening (20, 22) engage.

12. An applicator (10) according to claim 9, wherein at least one of said channels (20, 22) has an enlarged opening therein, and optionally wherein said enlarged opening is located at an end of a channel.

13. An applicator according to any of claims 9 to 11, wherein said enlarged opening (29) is shaped and configured to enable the circumferential groove of said support to pass therethrough.

14. An applicator according to any of claims 1 to 13, wherein said support (24) is generally ftusto-concical in shape, being tapered inwardly from an upper opening to a lower neck portion.

## Patentansprüche

1. Applikator (10) zum Aufbringen flüssiger Medikation auf ein Auge eines Benutzers, wobei der Applikator (10) einen Aufsatz und einen Schirmabschnitt (12) zum Aufbringen über dem Auge des Benutzers umfasst, wobei der Schirmabschnitt (12) eine Öffnung (20, 22) zum Aufnehmen des Aufsatzes (24) umfasst, wobei der Aufsatz so angeordnet ist, dass er die Abgabedüse einer Augentropfenbehälters aufnimmt, **dadurch gekennzeichnet, dass** die Medikation in den Aufsatz abgegeben werden kann und wobei die Öffnung (20, 22) so eingerichtet ist, dass sie die Medikation von dem Aufsatz (24) in das Auge des Benutzers leitet und freisetzt, und weiterhin **dadurch gekennzeichnet, dass** eine Öffnung in dem Aufsatz ein Mittel zum Regulieren des Flusses der Medikation, die von dem Aufsatz in das Auge des Benutzers läuft, enthält.

2. Applikator (10) nach Anspruch 1, wobei der Aufsatz (24) justierbar in der Öffnung montiert ist, so dass dessen Position im Verhältnis zu dem Auge des Benutzers gezielt justiert werden kann.

3. Applikator (10) nach Anspruch 1 oder 2, wobei die Öffnung (20, 22) einen oder mehrere längliche Kanäle (20, 22) umfassen kann, in denen der Aufsatz verschiebbar montiert sein kann.

4. Applikator (10) nach einem der Ansprüche 1 bis 3, wobei ein Mittel zum Immobilisieren des Aufsatzes in einer gewünschten Stellung in der Öffnung (20, 22) vorgesehen sein kann und gegebenenfalls wobei das Mittel ein Mittel zum Erzeugen von gegenseitiger Reibung zwischen dem Aufsatz und einer Berührungskante der Öffnung umfassen kann.

5. Applikator (10) nach einem der vorhergehenden Ansprüche, wobei die Öffnung (20, 22) in einem Plattenabschnitt, drehbar in oder dem Schirmabschnitt montiert, vorgesehen sein kann, so dass die Öffnung (20, 22) in Bezug auf eine im Allgemeinen zentrale, im Wesentlichen vertikale Achse gedreht werden kann, wodurch ermöglicht wird, dass eine Position des Aufsatzes (24) im Verhältnis zu dem Auge des Benutzers an einem beliebigen Punkt innerhalb eines Kreises, der von der Länge der Öffnung überstrichen wird, erreicht wird, und/oder wobei mindestens ein Halteband (16) zum Halten des Schirmabschnitts (12) über dem Auge des Benutzers vorgesehen ist, wobei das Halteband (16) mit dem Applikator (10) an im Wesentlichen diametrisch gegenüber liegenden Kantenteilen davon verbunden ist.

6. Applikator (10) nach Anspruch 5, wobei das Band (16) ein Halteband mit Gummizug umfasst, das als ein einziges Teil, das mit beiden Seiten des Schirmabschnitts verbunden ist, oder als zwei Teile mit einem Mittel zum lösbaren Verbinden der beiden Teile hergestellt ist.

7. Applikator (10) nach einem der Ansprüche 1 bis 6, wobei der Schirmabschnitt (12) aus einem starren oder halbstarren transparenten Material besteht.

8. Applikator (10) nach einem der Ansprüche 1 bis 7, wobei ein Rand (14) an dem Schirmabschnitt (12) vorgesehen ist, der zum Unterstützen einer bequemen Berührungsfläche zwischen dem Applikator (10) und der Höhle um das Auge des Benutzers eingerichtet ist.

9. Applikator (10) nach einem der Ansprüche 1 bis 8, wobei die Öffnung (20, 22) zwei längliche Kanäle (20, 22) in Kombination miteinander umfasst und gegebenenfalls wobei die Kanäle sich an jeweiligen Mittelpunkten dieser überschneiden und gegebenenfalls wobei die Kanäle im Wesentlichen orthogonal zueinander sind.

10. Applikator nach einem der Ansprüche 1 bis 9, wobei der Aufsatz (24) einen Halsabschnitt (30) aufweist, der passend in der Öffnung aufgenommen wird.

11. Applikator (10) nach Anspruch 10, wobei der Halsabschnitt (30) mit einer äußeren umlaufenden Kerbe versehen ist, mit der die Berührungskanten der Öffnung (20, 22) in Eingriff kommen.

12. Applikator (10) nach Anspruch 9, wobei mindestens einer der Kanäle (20, 22) eine vergrößerte Öffnung darin aufweist und gegebenenfalls wobei sich die vergrößerte Öffnung an einem Ende eines Kanals befindet.

13. Applikator nach einem der Ansprüche 9 bis 11, wobei die vergrößerte Öffnung (29) so geformt und eingerichtet ist, dass sie ermöglicht, dass die umlaufende Kerbe des Aufsatzes dort hindurch passt.

14. Applikator nach einem der Ansprüche 1 bis 13, wobei der Aufsatz (24) im Allgemeinen eine kegelstumpfartige Form aufweist, die von einer oberen Öffnung zu einem unteren Halsabschnitt nach innen verjüngt ist.

## Revendications

1. Applicateur (10) pour appliquer un médicament liquide à l'oeil d'un utilisateur, l'applicateur (10) comprenant un support et une portion protectrice (12) pour l'application sur l'oeil dudit utilisateur, dans lequel ladite portion protectrice (12) comprend une ouverture (20, 22) pour recevoir le support (24), le support étant agencé pour recevoir la buse de distribution d'un récipient de gouttes oculaires, **caractérisé en ce que** ledit médicament peut être distribué dans le support et dans lequel ladite ouverture (20, 22) est configurée pour guider et libérer le médicament depuis ledit support (24) dans l'oeil dudit utilisateur, et **caractérisé en outre en ce qu'**une ouverture dans ledit support contient un moyen de réguler l'écoulement de médicament passant dudit support à l'oeil de l'utilisateur.

2. Applicateur (10) selon la revendication 1, dans lequel ledit support (24) est monté de manière réglable dans ladite ouverture de sorte que son emplacement par rapport à l'oeil de l'utilisateur peut être réglé de manière sélective.

3. Applicateur (10) selon la revendication 1 ou 2, dans lequel ladite ouverture (20, 22) peut comprendre un ou plusieurs canaux allongés (20, 22) dans lesquels le support peut être monté à coulissement.

4. Applicateur (10) selon l'une quelconque des revendications 1 à 3, dans lequel un moyen peut être prévu pour immobiliser le support en un emplacement souhaité au sein de ladite ouverture (20, 22), et dans lequel ledit moyen peut comprendre en option un moyen pour générer une friction mutuelle entre ledit support et un bord de contact de ladite ouverture.

5. Applicateur (10) selon l'une quelconque des revendications précédentes, dans lequel ladite ouverture (20, 22) peut être prévue dans une portion de plaque, montée à rotation dans ou ladite portion protectrice, de sorte que ladite ouverture (20, 22) peut être tournée par rapport à un axe généralement central, essentiellement vertical permettant l'emplacement du support (24) par rapport à l'oeil de l'utilisateur en tout point au sein d'un cercle balayé par la longueur de l'ouverture devant être réalisée, et/ou dans lequel au moins une sangle de retenue (16) est prévue pour retenir ladite portion protectrice (12) sur l'oeil dudit utilisateur, ladite sangle de retenue (16) étant connectée à l'applicateur (10) au niveau de parties de bord de celui-ci essentiellement diamétralement opposées.

6. Applicateur (10) selon la revendication 5, dans lequel ladite sangle (16) comprend une sangle de retenue élastiquée réalisée en tant que morceau unique connecté de chaque côté de la portion protectrice ou en deux morceaux avec un moyen pour relier les deux de manière à ce qu'ils puissent être libérés.

7. Applicateur selon l'une quelconque des revendications 1 à 6, dans lequel ladite portion protectrice (12) est constituée d'un matériau transparent rigide ou semi-rigide.

8. Applicateur (10) selon l'une quelconque des revendications 1 à 7, dans lequel un bord (14) est prévu sur ladite portion protectrice (12), configuré pour faciliter une interface confortable entre ledit applicateur (10) et l'orbite autour de l'oeil dudit utilisateur.

9. Applicateur (10) selon l'une quelconque des revendications 1 à 8, dans lequel ladite ouverture (20, 22) comprend deux canaux allongés (20, 22) en communication l'un avec l'autre, et dans lequel lesdits canaux se coupent en option au niveau de moitiés respectives de ceux-ci ; et dans lequel lesdits canaux sont en option essentiellement orthogonaux l'un par rapport à l'autre.

10. Applicateur selon l'une quelconque des revendications 1 à 9, dans lequel ledit support (24) possède une portion de col (30) qui est reçue de manière appropriée dans ladite ouverture.

11. Applicateur (10) selon la revendication 10, dans lequel ladite portion de col (30) est pourvue d'une rainure externe circonférentielle avec laquelle les bords de contact de l'ouverture (20, 22) s'engagent.

12. Applicateur (10) selon la revendication 9, dans lequel au moins un desdits canaux (20, 22) possède une ouverture agrandie en son sein, et dans lequel ladite ouverture agrandie est située en option à une extrémité d'un canal.

13. Applicateur selon l'une quelconque des revendications 9 à 11, dans lequel ladite ouverture agrandie (29) est formée et configurée pour permettre à la rainure circonférentielle dudit support de passer à travers elle.

14. Applicateur selon l'une quelconque des revendications 1 à 13, dans lequel ledit support (24) est de forme généralement tronconique, étant amincie vers l'intérieur d'une ouverture supérieure vers une portion de col inférieure.
